# EUROPÄISCHE PATENTSCHRIFT

(11) **EP 1 581 170 B1**
(45) Veröffentlichungstag und Bekanntmachung des Hinweises auf die Patenterteilung: **24.01.2018**
(21) Anmeldenummer: 03789353.4
(22) Anmeldetag: 19.12.2003
(51) Int. Cl.: A61K 8/37, A61K 8/34, A61K 8/33

(54) **WACHSDISPERSIONEN**
WAX DISPERSIONS
DISPERSIONS DE CIRE

(30) Priorität: 08.01.2003 DE 10300506
(43) Veröffentlichungstag der Anmeldung: 05.10.2005
(73) Patentinhaber: Cognis IP Management GmbH, 40589 Dusseldorf (DE)
(72) Erfinder: BRÜNING, Stefan, Philadelphia, PA 19118 (US); CAPITO, Marco, 40721 Hilden (DE); SPÖRER, Roland, 41352 Kleinenbroich/Glehn (DE); ANSMANN, Achim, 40699 Erkrath (DE); LEONARD, Mark, Bexley Kent DA5 1AZ (GB)
(74) Vertreter: BASF IP Association
(86) Internationale Anmeldenummer: PCT/EP2003/014596
(87) Internationale Veröffentlichungsnummer: WO 2004/062630

(56) Entgegenhaltungen:
- EP-A- 0 394 078
- WO-A-00/10510
- WO-A-98/20840
- WO-A-02/056839
- WO-A-03/037292
- DE-A- 4 411 557
- DE-A- 19 737 737
- DE-A- 19 837 191
- US-A- 5 478 555
- US-A1- 2002 055 560
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631-662, XP009102859, ISSN: 0193-2691, DOI: 10.1081/DIS-120015368

## Beschreibung

### Gebiet der Erfindung

Gegenstand der Erfindung sind spezielle Wachsdispersionen, die als Grundlage für kosmetische Mittel verwendet werden können sowie insbesondere zur Imprägnierung und Benetzung von Gebrauchs- und Hygienetüchern, die zur Körperreinigung und -pflege eingesetzt werden.

### Stand der Technik

Unter dem Oberbegriff "Papier" werden ca. 3000 verschiedene Sorten und Artikel verstanden, deren Beschaffenheit und Anwendungsgebiete sich zum Teil erheblich unterscheiden können. Zur Herstellung von Papier benötigt man eine Reihe von Zusatzstoffen, von denen Füllstoffe (z.B. Kreide oder Kaolin) und Bindemittel (z.B. Stärke) zu den wichtigsten zählen. Für den Bereich der Tissue- und Hygienepapiere, die in engeren Kontakt mit der menschlichen Haut gebracht werden, besteht ein besonderes Bedürfnis nach einem angenehmen Weichgriff, der dem Papier üblicherweise durch eine sorgfältige Auswahl der Faserstoffe und insbesondere einen hohen Anteil an frischem Holzschliff oder Cellulose verliehen wird. Im Hinblick auf die Wirtschaftlichkeit der Papierherstellung sowie aus ökologischer Sicht, ist es jedoch wünschenswert, möglichst hohe Anteile an qualitativ minderwertigerem Altpapier mitzuverwenden. Dies hat jedoch zur Folge, daß der Weichgriff des Papiers signifikant verschlechtert wird, was von den Anwendern als störend empfunden wird und insbesondere bei häufigem Gebrauch auch zu Hautirritationen führen kann.

In der Vergangenheit hat es daher nicht an Versuchen gemangelt, Papier durch Tränken, Beschichten oder andere Oberflächenbehandlung so zu modifizieren, daß eine angenehmere Sensorik resultiert. Hierfür werden spezielle Lotionen und Emulsionen entwickelt, die sich einerseits leicht auf das Papier auftragen lassen, anderseits dessen Struktur nicht negativ beeinflussen. Um den Weichgriff zu verbessern, werden häufig Niotenside oder eine Kombination aus Nio- und Aniontensiden verwendet. Auch Polysiloxane und kationische Polymere werden für diesen Zweck eingesetzt.

In der internationalen Patentanmeldung WO98/20840 werden Dialkylether als Wachskörper aufgrund des brillianten Perlglanzes, der avivierenden Eigenschaften sowie der kosmetischen Verträglichkeit in Haarshampoos empfohlen. Auch die WO 02/056839 offenbart Perlglanzkonzentrate für kosmetische Anwendungen wie Haarshampoos auf Basis von Wachsen, die sich durch amorphe und kristalline Anteile auszeichnen. Wässrige Dispersionen, die Wachskörper wie Fettcarbonate oder Fettether und Emulgatoren enthalten, sind gemäß der WO 00/10510 als Konsistenzgeber zur kalten Herstellung von O/W-Emulsionen geeignet. Die DE 44 11 557 wiederum beschreibt ein Verfahren zur Herstellung von Mikroemulsionen enthaltend als Ölkomponente Dialkylether sowie spezielle Emulgatormischungen, wobei die Mikroemulsionen beispielsweise als Badeöle verwendet werden können. Des Weiteren sind aus der US 5 478 555 kosmetische Make-up-Zusammensetzungen auf Basis von Silikonwachsen und speziellen Acrylat-Polymeren bekannt. Die EP0394078 wiederum offenbart kosmetische Zusammensetzungen für Haare enthaltend Mikrodispersionen mit Wachsen kleiner 500 nm und Schmelzpunkten oberhalb von 60 °C und Emulgiermittel in bestimmten Mengenverhältnissen. Bevorzugt sind Carnaubawachs, Candelillawachs oder deren Mischungen.

Schließlich beschreibt die Deutsche Offenlegungsschrift DE 19837191 wäßrige Zubereitungen zur Behandlung von Haut, wobei Wirkstoffe wie Vitamine als Bestandteile in feinen Wachspartikeln mit Teilchengrößen unter 500 nm enthalten sind.

Trockene Kosmetiktücher mit punktförmigen Vertiefungen werden in der internationalen Anmeldung WO 03/037292 beschrieben. Die strukturierte Oberfläche in Kombination mit Tensidformulierungen enthaltend feindispergierte Wachskörper führen durch geringen mechanischen Einfluß zu einem voluminösem Schaum und hinterlassen ein angenehmes Hautgefühl.

Gegenstand der internationalen Patentanmeldung WO 95/35411 sind Tissuepapiere, die mit Avivagemitteln beschichtet werden, welche 20 bis 80 Gew.-% eines wasserfreien Emollients (Mineralöle, Fettsäureester, Fettalkoholethoxylate, Fettsäureethoxylate, Fettalkohole und deren Mischungen), 5 bis 95 Gew.-% eines das Emollient "immobilisierenden Agens" (Fettalkohole, Fettsäuren oder Fettalkoholethoxylate mit jeweils 12 bis 22 Kohlenstoffatomen im Fettrest) sowie 1 bis 50 Gew.-% Tenside mit einem HLB-Wert von vorzugsweise 4 bis 20 enthalten. Die in der Schrift aufgeführten Ausführungsbeispiele enthalten als Emollient ausnahmslos Petrolatum. Die internationale Patentanmeldung WO 95/35412 offenbart ähnliche Tissuepapiere, wobei als Softener wasserfreie Mischungen von (a) Mineralölen, (b) Fettalkoholen oder Fettsäuren und (c) Fettalkoholethoxylaten zum Einsatz kommen. Gegenstand der internationalen Patentanmeldung WO 95/16824 sind Avivagemittel für Tissuepapiere, die Mineralöl, Fettalkoholethoxylate und nichtionische Tenside (Sorbitanester, Glucamide) enthalten. Des weiteren werden in der internationalen Patentanmeldung WO 97/30216 (Kaysersberg) flüssige Avivagemittel für Papiertaschentücher auf Basis von langkettigen, gesättigten Fettalkoholen und Wachsestern mit insgesamt wenigstens 24 Kohlenstoffatomen beschrieben, die einen sehr hohen Wasseranteil enthalten. Die Patentschrift DE 33 09 530 beschreibt hygienische Absorptionsvorlagen, die mit Glyceriden und/oder Partialglyceriden der Kokosfettsäuren belegt sind. Beschichtungen für Hygieneprodukte werden auch in R. E. Mathis, Nonwovens World 1999, Seiten 59 - 65 beschrieben.

Vom anwendungstechnischen Standpunkt ist insbesondere die Sensorik der behandelten Papiere und Tissues nach wie vor verbesserungswürdig. Die gegenwärtig verwendeten Beschichtungen hinterlassen ein zu wenig pflegendes und oft ein zu belastendes, fettiges Hautgefühl und zeichnen sich zum Teil durch eine zu langsame Wirkstofffreigabe aus. Insbesondere auf dem Gebiet der Baby-Hygiene ist eine effektive Wirkstoff-Freisetzung, eine verbesserte Pflegeleistung und Sensorik von großer Bedeutung. Auch die Langzeitstabiltät der beschichteten Wipes bei Lagerung ist verbesserungswürdig. Die Beschichtung sollte sich bei längerer Lagerung nicht auf dem Boden des Vorratsgefäßes absetzen. Vom fertigungstechnischen Standpunkt ist die Auftragbarkeit der Zusammensetzungen verbesserungswürdig. Insbesondere ist es wünschenswert, Beschichtungen zu entwickeln, die kalt applizierbar sind und somit aus fertigungstechnischer Sicht kostengünstiger und auch weniger geruchsbelästigend sind.

Der Erfindung lag die Aufgabe zugrunde, Zusammensetzungen zur Beschichtung von Tissuepapieren, zur Herstellung von Wet Wipes und Dry Wipes zur Verfügung zu stellen, die sich durch eine verbesserte Sensorik auszeichnen, insbesondere ein pflegendes, nichtbelastendes und nicht fettiges Hautgefühl. Ein weiterer Aspekt der Aufgabe war es, Zusammensetzungen zur Verfügung zu stellen, die besonders leicht auf Unterlagen appliziert werden können. Die beschichteten Papiere/Wipes sollten ausgezeichnete pflegende Eigenschaften aufweisen, eine effiziente Wirkstofffreisetzung gewährleisten und sich durch besondere Milde und Hautverträglichkeit auszeichnen. Des weiteren sollten nur leicht biologisch abbaubare Hilfsstoffe Verwendung finden und die Zubereitungen leicht in das Tissue eindringen, sich homogen verteilen, und leicht verarbeitbar sein.

### Beschreibung der Erfindung

Es wurde gefunden, daß Wachsdispersionen mit einem Gehalt an speziellen Öl- oder Wachskörpern und einer Teilchengröße im Mikrometerbereich ausgezeichnete sensorische und pflegende Eigenschaften aufweisen, sehr leicht auf Unterlagen applizierbar sind und daher für die Imprägnierung von Papieren und Wipes für den Körperpflegebereich besonders gut geeignet sind.

Gegenstand der vorliegenden Anmeldung sind daher Wachsdispersionen mit einer durchschnittlichen Partikelgröße (mittels Fraunhofer-Beugung) von 0,5 bis 100 µm umfassend
(a) 10-75 Gew.-% bezogen auf die Wachsdispersion einer Wachsphase, die im Bereich oberhalb 25 °C und bis zu 50 °C schmilzt, die wenigstens eine Öl- oder Wachskomponente ausgewählt aus den Dialkyl(en)ethern, Dialkyl(en)carbonaten, Dicarbonsäuren oder Hydroxyfettalkoholen oder einem beliebigen Gemisch dieser Substanzen und wenigstens einen Emulgator enthält, und
(b) eine Wasserphase.

In einer bevorzugten Ausführungsform enthalten die erfindungsgemäßen Wachsdispersionen (a) 10 - 75 Gew.-% einer Wachsphase, die im Bereich oberhalb 25 °C und bis zu 50 °C schmilzt, die wenigstens eine Öl- oder Wachskomponente ausgewählt aus den Dialkyl(en)ethern, Dialkyl(en)carbonaten, Dicarbonsäuren oder Hydroxyfettalkoholen oder einem beliebigen Gemisch dieser Substanzen und wenigstens einen Emulgator umfaßt und 25 - 90 Gew.% einer wässrigen Phase, bezogen auf die Gesamtzusammensetzung. Erfindungsgemäß bevorzugte Wachsdispersionen enthalten 10 - 30 Gew.-% der obigen Wachsphase; besonders bevorzugt ist eine Gehalt von 10 - 25 Gew.-% der Wachsphase bezogen auf die Wachsdispersion.

Die erfindungsgemäßen Wachsdispersionen lassen sich im Fertigungsprozeß in feindisperser Verteilung, kalt verarbeitbar und einfacher als eine Schmelze aus Wachsen auf Unterlagen, wie Papiere und Tissues, aufbringen. Sie können sowohl in Form von Konzentraten mit einem Wassergehalt von nur 25 Gew.-% appliziert werden, als auch hochverdünnt, z. B. in einer Konzentration von nur 1 Gew.-%, appliziert werden. Durch die Kombination mit speziellen Öl- und Wachskörpern, ausgewählt aus den Dialkyl(en)ethern, Dialkyl(en)carbonaten, Dicarbonsäuren oder Hydroxyfettalkoholen bzw. Gemischen davon sind die erfindungsgemäßen Wachsdispersionen hinsichtlich der sensorischen Eigenschaften optimiert. Insbesondere werden sie als pflegend, weniger belastend und weniger fettend empfunden als vergleichbare Zusammensetzungen des Standes der Technik und vermitteln ein eher trockenes Hautgefühl bei ausgezeichneten pflegenden Eigenschaften.

Ein weiterer Gegenstand der Erfindung ist die Verwendung der erfindungsgemäßen Wachsdispersion zur Herstellung von Körperpflegemitteln. Dies erfolgt durch Zugabe der in der Kosmetik üblichen Hilfs- und Zusatzstoffe und Wasser.

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen üblicherweise oberhalb von 25° C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar sind wachsartige Zusammensetzungen oder Wachsphasen, die sich durch einen Schmelzpunkt auszeichnen, der oberhalb von 25 °C liegt, die sich also erst oberhalb von 25 °C vollständig verflüssigen. Sie können also noch einen gewissen Anteil an flüssigen Bestandteilen oder niedriger schmelzenden Komponenten enthalten.

Die Wachsphase der erfindungsgemäßen Zusammensetzungen enthält weniger als 10 Gew.-% Wasser, vorzugsweise liegt der Wassergehalt bei weniger als 6 Gew.-%, und insbesondere weniger als 3 Gew.-%. In einer besonders bevorzugten Ausführungsform ist die Wachsphase wasserfrei. Unter wasserfrei im Sinne der Erfindung ist zu verstehen, daß die Wachsphase lediglich rohstoffbedingt einen geringen Wasseranteil enthalten kann, daß aber kein zusätzliches Wasser zugegeben wird. Beim Verarbeitungsprozeß und Applikation der Zusammensetzung auf die Wipes erlaubt dies Nachbehandlungsschritte der Wipes mit wässrig/tensidischen Lösungen, ohne daß sich die Wachsdispersion ablöst. Die Wasserphase kann wasserlösliche Wirkstoffe, z. B. Harnstoff und Hydantoin, wasserlösliche oder wasserquellbare Polymere, Feuchthaltemittel, etc. enthalten.

Die Wachsphase der erfindungsgemäßen Wachsdispersionen läßt sich auch ausschließlich aus Dialkyl(en)ethern, Dialkyl(en)carbonaten, Dicarbonsäuren oder Hydroxyfettalkoholen oder einem beliebigen Gemisch dieser Substanzen mit wachsartiger Konsistenz formulieren, enthalten aber vorzugsweise - und je nach Anforderungsprofil - weitere wachsartige Lipidkomponenten und Öle. Wesentlich ist, daß die Wachsphase oberhalb 25°C und bis zu 50°C schmilzt. Es können also erfindungsgemäß auch flüssige Dialkyl(en)ether, Dialkyl(en)carbonate, Dicarbonsäuren oder Hydroxyfettalkohole eingearbeitet werden, solange die Wachsphase den geforderten Schmelzpunkt von höher als 25 °C aufweist. Eine bevorzugte Ausführungsform der erfindungsgemäßen Wachsdispersion enthält eine Wachsphase mit einem Schmelzpunkt im Bereich von 35 - 50 °C, vorzugsweise 35 - 45 °C und ganz besonders bevorzugt 37 - 42 °C. Hierdurch wird gewährleistet, daß die Wachsphase nach Beschichtung der Wipes fein-partikulär vorliegt und sich erst bei Applikation bei Körpertemperatur verflüssigt. Mit derartigen Wachsdispersionen beschichtete Tücher und Wipes sind besonders lagerstabil, eine Vermischung der Phasen wird vermieden. Erst bei Applikation der Tücher auf der Haut schmilzt die fein-partikuläre Wachsphase.

Erfindungsgemäß besonders vorteilhaft sind Wachsdispersionen, die Partikel mit einer durchschnittlichen Teilchengröße von 1 - 50 µm und insbesondere 5 - 30 µm enthalten.

Die Dialkyl(en)ether können symmetrisch oder unsymmetrisch, verzweigt oder unverzweigt, gesättigt oder ungesättigt sein. Erfindungsgemäß bevorzugt geeignet sind wachsartige, gesättigte C₁₆-C₃₀-Dialkylether, insbesondere C₁₆-C₂₄-Dialkylether. Besonders bevorzugt sind C₁₆-C₂₀-Dialkylether, und insbesondere bevorzugt geeignet sind Distearylether und Dibehenylether. Erfindungsgemäß können auch kürzerkettige Dialkylether eingearbeitet werden, wie beispielsweise Di-n-octylether, Di-(2-ethylhexyl)-ether, Laurylmethylether oder Octylbutylether, Didodecylether, solange die Wachsphase den geforderten Schmelzpunk hat. Die Dialkyl(en)ether lassen sich aus Fettalkoholen in Gegenwart saurer Katalysatoren nach allgemein bekannten Verfahren des Standes der Technik herstellen, z. B. DE 195 11 668 A1 und DE 198 31 705 A1 sowie DE 199 43 585. Typische Beispiele für derartige Ether sind Produkte, die durch Veretherung von Capronalkohol, Caprylalkohol, 2-Ethylhexylalkohol, Caprinalkohol, Laurylalkohol, Myristylalkohol, Cetylalkohol, Palmoleylalkohol, Stearylalkohol, Isostearylalkohol, Elaidylalkohol, Petroselinylalkohol, Linolylalkohol, Linolenylalkohol, Oleylalkohol, Rizinolalkohol, Elaeostearylalkohol, Arachidylalkohol, Gadoleylalkohol, Behenylalkohol, Erucylalkohol und Brassidylalkohol, Guerbetalkoholen, sowie deren technische Mischungen, die z.B. bei der Hochdruckhydrierung von technischen Methylestern auf Basis von Fetten und Ölen anfallen, gewonnenen werden. Bevorzugt geeignet sind bei 25 °C feste Dialkyl(en)ether.

Die Dialkyl(en)carbonate können symmetrisch oder unsymmetrisch, verzweigt oder unverzweigt, gesättigt oder ungesättigt sein. Unter den Dialkylcarbonaten sind wachsartige, lineare oder verzweigte, gesättigte oder ungesättigte C₁₄-C₃₀-Dialkyl(en)carbonate erfindungsgemäß bevorzugt. Besonders bevorzugt sind C₁₆-C₂₄-Dialkyl(en)carbonate und unter diesen gesättigte, unverzweigte C₁₆-C₂₂-Dialkylcarbonate. Besonders bevorzugt geeignet ist Distearylcarbonat. Aber auch flüssige Dialkyl(en)carbonate, wie z. B. Dihexyl-, Dioctyl-, Di-(2-ethylhexyl)- oder Dioleylcarbonat, sind erfindungsgemäß einsetzbar, solange die Wachsphase der Wachspdispersion den geforderten Schmelzpunkt aufweist. Die Verbindungen lassen sich durch Umesterung von Dimethyl- oder Diethylcarbonat mit den entsprechenden Hydroxyverbindungen nach Verfahren des Standes der Technik herstellen; eine Übersicht hierzu findet sich in Chem.Rev. 96, 951 (1996**)**. Typische Beispiele für Dialkyl(en)carbonate sind Umesterungsprodukte von Dimethyl- und/oder Diethylcarbonat mit Capronalkohol, Caprylalkohol, 2-Ethylhexylalkohol, Caprinalkohol, Laurylalkohol, Myristylalkohol, Cetylalkohol, Palmoleylalkohol, Stearylalkohol, Isostearylalkohol, Elaidylalkohol, Petroselinylalkohol, Linolylalkohol, Linolenylalkohol, Oleylalkohol, Rizinolalkohol, Elaeostearylalkohol, Arachidylalkohol, Gadoleylalkohol, Behenylalkohol, Erucylalkohol und Brassidylalkohol, Guerbetalkoholen, sowie deren technische Mischungen, die z.B. bei der Hochdruckhydrierung von technischen Methylestern auf Basis von Fetten und Ölen anfallen. Bevorzugt geeignet sind bei 25 °C feste Dialkyl(en)carbonate.

Als Dicarbonsäuren lassen sich erfindungsgemäß C₉-C₃₄-Dicarbonsäuren einsetzen. Hierzu zählen z.B. Octadecandisäure, Tetratridecansäure, etc. Erfindungsgemäß bevorzugt geeignet ist Azelainsäure, eine C₉-Dicarbonsäure.

Unter den Hydroxyfettalkoholen sind gesättigte oder ungesättigte, verzweigte oder unverzweigte Verbindungen geeignet. C₁₂-C₃₀-Fettalkohole sind bevorzugt geeignet, wobei die Stellung des Hydroxy-Substituenten vom Syntheseweg und den eingesetzten Edukten abhängt. Hierzu zählen z. B. 1,10-Decandiol (Speziol® 10/2), 1,2-Hexadecandiol, 12-Hydroxystearylalkohol oder Hydroxy-Guerbetalkohole. Erfindungsgemäß bevorzugt geeignet sind bei 25 °C feste Hydroxyfettalkohole, obgleich auch flüssige einsetzbar sind, solange die Wachsphase den geforderten Schmelzpunkt aufweist. Besonders bevorzugt geeignet ist 12-Hydroxystearylalkohol, der von der Cognis France S.A. unter der Bezeichnung Speziol® 18/2 vermarktet wird. 1,2-Hexadecandiol erhält man z. B. durch Ringöffnung des entsprechenden α-Epoxids.

Die Dialkylether, Dialkylcarbonate und Dicarbonsäuren sowie Hydroxyalkohole sind bezogen auf die Gesamtzusammensetzung vorzugsweise in einer Menge von insgesamt 0,1 - 30 Gew.-%, besonders bevorzugt 0,5 - 20 Gew.-% und insbesondere 0,5 - 10 Gew.-% enthalten.

Die erfindungsgemäßen Zusammensetzungen sind praktisch geruchsfrei, ökotoxikologisch unbedenklich und leicht biologisch abbaubar. Sie eignen sich als fetthaltige, milde kosmetische Mittel und können auch als Basis in alle kosmetischen Mittel zur Körperpflege und -reinigung wie Cremes, Lotionen, sprühbare Emulsionen, Sonnenschutzmittel, Antitranspirantien etc. eingearbeitet werden. Sie lassen sich als Pflegekomponente auf Tissues, Papiere und Wipes applizieren, die ihren Einsatz im Bereich der Hygiene und Pflege finden (Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes).

### Emulgator

Durch den Zusatz der Emulgatoren wird die Stabilität der fein-partikulären Wachsdispersion erhöht. Als Emulgatoren geeignet sind ionische und nicht-ionische Emulgatoren, wobei nicht-ionische Emulgatoren erfindungsgemäß bevorzugt sind. Des weiteren lassen sich geringe Mengen wasserlöslicher Substanzen und Wirkstoffe, Wasser sowie Feuchthaltemittel einarbeiten.

Die in einer bevorzugten Ausführungsform der Erfindung enthaltenen nicht-ionischen Emulgatoren zeichnen sich durch ihre Hautfreundlichkeit und Milde sowie ihre ökotoxologisch guten Eigenschaften aus. Sie sind darüber hinaus zur Stabilisierung der fein-partikulären Wachsdispersion besonders gut geeignet. Durch Verwendung einer Kombination nicht-ionischer W/O- und O/W-Emulgatoren erhält man Zusammensetzungen mit verbesserter Stabilität. Die erfindungsgemäßen Wachsdispersionen enthalten den/die Emulgator(en) in einer Menge von 0,1 bis 10 Gew.-%, vorzugsweise 0,5 bis 7 Gew.-% und insbesondere 1 bis 5 Gew.-% bezogen auf das Gesamtgewicht der Wachsdispersion.

### Nicht-ionische Emulgatoren

Zur Gruppe der nicht-ionischen Emulgatoren gehören:
(1) Anlagerungsprodukte von 2 bis 50 Mol Ethylenoxid und/oder 1 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.
(2) C_{12/18}-Fettsäuremono- und -diester von Anlagerungsprodukten von 1 bis 50 Mol Ethylenoxid an Glycerin.
(3) Ethylenoxidanlagerungsprodukte von Glycerinmono- und -diestern; Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z. B. Polyolpoly-12-hydroxystearate, Polyglycerinpolyricinoleat, Polyglycerindiisostearat oder Polyglycerindimerat. Ebenfalls geeignet sind Gemische von Verbindungen aus mehreren dieser Substanzklassen.
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆-C₂₂-Fettsäuren, Ricinolsäure sowie 12-Hydroxystearinsäure und Glycerin, Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose), oder Mischester wie z. B. Glycerylstearatcitrat und Glycerylstearatlactat.
(9) Polysiloxan-Polyalkyl-Polyether-Copolymere bzw. entsprechende Derivate.
(10) Mischester aus Pentaerythrit, Fettsäuren, Citronensäure und Fettalkohol gemäß und/oder Mischester von Fettsäuren mit 6 bis 22 Kohlenstoffatomen, Methylglucose und Polyolen, vorzugsweise Glycerin oder Polyglycerin.
(11) Polyalkylenglykole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. C_{12/18}-Fettsäuremono- und -diester von Anlagerungsprodukten von Ethylenoxid an Glycerin sind als Rückfettungsmittel für kosmetische Zubereitungen bekannt.

Erfindungsgemäß bevorzugt geeignete, sehr milde Emulgatoren sind Polyolpoly-12-hydroxystearate und Abmischungen davon, welche beispielsweise unter den Marken "Dehymuls® PGPH" (W/O-Emulgator) oder "Eumulgin® VL 75" (Abmischung mit Coco Glucosides im Gewichtsverhältnis 1:1, O/W-Emulgator) oder Dehymuls® SBL (W/O-Emulgator) von der Cognis Deutschland GmbH & Co. KG vertrieben werden. In diesem Zusammenhang sei insbesondere auf das Europäische Patent EP 0 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Als lipophile W/O-Emulgatoren eignen sich prinzipiell Emulgatoren mit einem HLB-Wert von 1 bis 8, die in zahlreichen Tabellenwerken zusammengefasst und dem Fachmann bekannt sind. Einige dieser Emulgatoren sind beispielsweise in Kirk-Othmer, "Encyclopedia of Chemical Technology", 3. Aufl., 1979, Band 8, Seite 913, aufgelistet. Für ethoxylierte Produkte läßt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, wie beispielsweise Partialester des Pentaerythrits oder Zuckerester, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Als Emulgatoren geeignet sind auch die entsprechenden Anlagerungsprodukte von Ethylenoxid.

Im Falle der Einarbeitung wasserlöslicher Wirkstoffe und/oder geringer Mengen Wasser kann es weiterhin vorteilhaft sein, zusätzlich wenigstens einen Emulgator aus der Gruppe nicht-ionischer O/W-Emulgatoren (HLB-Wert: 8 - 18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10 - 20 Ethylenoxid-Einheiten für O/W-Emulgatoren und 20 - 40 Ethylenoxid-Einheiten für sogenannte Solubilisatoren. Erfindungsgemäß vorteilhaft als O/W-Emulgatoren sind Ceteareth-12 und PEG-20 Stearat. Als Solubilisatoren geeignet sind Eumulgin® HRE 40 (INCI: PEG-40 Hydrogenated Castor Oil), Eumulgin® HRE 60 (INCI: PEG-60 Hydrogenated Castor Oil), Eumulgin® L (INCI: PPG-1-PEG-9 Laurylglycolether), sowie Eumulgin® SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. C₈-C₂₂-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare® zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß vorteilhaft einsetzbar ist z. B. ein Produkt, das unter der Bezeichnung Emulgade® PL 68/50 von der Cognis Deutschland GmbH vertrieben und ein 1:1-Gemisch aus Alkylpolyglucosiden und Fettalkoholen darstellt. Erfindungsgemäß bevorzugt geeignet ist auch ein Gemisch aus Lauryl Glucoside, Polyglyceryl-2-Dipolyhydroxystearate, Glycerin und Wasser, das unter der Bezeichnung Eumulgin® VL 75 im Handel ist.

### Weitere Tenside/Emulgatoren

Die erfindungsgemäßen Wachsdispersionen können je nach Verwendungszweck der Wipes und Tissues weiterhin anionische, zwitterionische, amphotere oder kationische Tenside enthalten.

Erfindungsgemäß geeignet ist auch der Zusatz anionischer Tenside/Emulgatoren zur Wachsdispersion. Sie sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze. Erfindungsgemäß bevorzugt geeignet unter den anionischen Tensiden/Emulgatoren sind Alkylsulfate und Alkylethersulfate.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylamino-propyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder - SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Als kationische Tenside sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex® vertriebenen Dialkylammoniummethosulfate und Methylhydroxyalkyldialkoyloxyalkylammoniummethosulfate und die entsprechenden Produkte der Dehyquart®-Reihe, als kationische Tenside eingesetzt werden. Unter der Bezeichnung "Esterquats" werden im allgemeinen quaternierte Fettsäuretriethanolaminestersalze verstanden. Sie können den erfindungsgemäßen Zusammensetzungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

### Weitere wachsartige Lipidkomponenten

In einer weiteren bevorzugten Ausführungsform enthält die Wachsphase der Wachsdispersion wenigstens eine weitere wachsartige Lipidkomponente. Durch den Zusatz weiterer wachsartiger Lipidkomponenten läßt sich die Sensorik sowie die Stabilität der Wachsdispersion weiter optimieren und dem Anforderungsprofil anpassen. Unter "wachsartig" werden Verbindungen verstanden, die von wachsartiger Konsistenz sind (s.o.) und einen Schmelzpunkt oberhalb 25 °C aufweisen.

Als weitere Lipidkomponenten (Definition vgl.: CD Römpp Chemie Lexikon - Version 1.0, Stuttgart/New York: Georg Thieme Verlag 1995) können erfindungsgemäß alle Fette und fettähnlichen Substanzen mit wachsartiger Konsistenz eingesetzt werden. Hierzu gehören u.a. Fette (Triglyceride), Mono- und Diglyceride, Wachse, Fett- und Wachsalkohole, Fettsäuren, Ester von Fettalkoholen sowie Fettsäureamide oder beliebige Gemische dieser Substanzen. Sie können in den erfindungsgemäßen Zusammensetzungen in einer Gesamtmenge von 0,1 - 45 Gew.-% enthalten sein. Bevorzugt ist eine Gesamtmenge von 5 - 30 Gew.-% und insbesondere 10 - 25 Gew.-% bezogen auf die gesamte Wachsdispersion.

### Fette

Unter Fetten versteht man Triacylglycerine, also die Dreifachester von Fettsäuren mit Glycerin. Unter den Triacylglycerinen sind jene als Lipidkomponente bevorzugt, die sich im Bereich von 35 - 50 °C, vorzugsweise 35 - 45 °C und insbesondere 37 - 42 °C verflüssigen. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind sogenannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C₁₂-C₆₀-Fettsäuren und insbesondere C₁₂-C₃₆-Fettsäuren. Hierzu zählt gehärtetes Rizinusöl, ein Dreifachester aus Glycerin und einer Hydroxystearinsäure, der beispielsweise unter der Bezeichnung Cutina® HR im Handel ist. Ebenso geeignet sind Glycerintristearat, Glycerintribehenat (z. B. Syncrowax® HRC), Glycerintripalmitat oder die unter der Bezeichnung Syncrowax® HGLC bekannten Triglycerid-Gemische, mit der Vorgabe, daß der Schmelzpunkt der Wachsphase oberhalb von 25 °C, und vorzugsweise bei 35 - 50 °C liegt.

Als Lipidkomponenten sind neben den Triglyceriden auch Mono- und Diglyceride bzw. Mischungen der Glyceride einsetzbar. Zu den erfindungsgemäß bevorzugten Glyceridgemischen zählen die von der Cognis Deutschland GmbH & Co. KG vermarkteten Produkte Novata® AB und Novata® B (Gemisch aus C₁₂-C₁₈-Mono-, Di- und Triglyceriden) sowie Cutina® MD oder Cutina® GMS (Glycerylstearat). Das Glycerid(gemisch) kann in einer Menge von 0,1 - 45 Gew.-% bezogen auf die Wachsdispersion enthalten sein, bevorzugt sind Mengen von 0,1 - 15 Gew.-% und insbesondere 1 - 12 Gew.-% bezogen auf die Wachsdispersion.

Mischester sowie Mischungen aus Mono-, Di- und Triglyceriden sind erfindungsgemäß bevorzugt geeignet, da sie eine geringere Neigung zur Kristallisation zeigen und somit die Performance der erfindungsgemäßen Zusammensetzung verbessern.

### Fettalkohole und Fettsäuren

Zu den erfindungsgemäß einsetzbaren Fettalkoholen mit wachsartiger Konsistenz zählen u.a. die C₁₂-C₆₀-Fettalkohole, insbesondere die C₂-C₂₄-Fettalkohole, die aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol sowie wachsartige Guerbetalkohole. Erfindungsgemäß bevorzugt sind gesättigte, verzweigte oder unverzweigte Fettalkohole. Erfindungsgemäß einsetzbar sind auch wachsartige Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch wachsartige synthetische Alkohole, z. B. die linearen, geradzahligen Fettalkohole der Ziegler-Synthese (Alfole®) oder die teilweise verzweigten Alkohole aus der Oxosynthese (Dobanole®) verwendet werden. Erfindungsgemäß geeignet sind C₁₄-C₁₈-Fettalkohole, die beispielsweise von der Cognis Deutschland GmbH & Co. KG unter der Bezeichnung Lanette® 16 (C₁₆-Alkohol), Lanette® 14 (C₁₄-Alkohol), Lanette® O (C₁₆/C₁₈-Alkohol) und Lanette® 22 (C₁₈/C₂₂-Alkohol) vermarktet werden. Fettalkohole verleihen den Zusammensetzungen ein trockeneres Hautgefühl als Triglyceride und sind daher bevorzugt geeignet. Der/die Fettalkohole können in einer Menge von 0,1 - 45 Gew.-% bezogen auf die Wachsdispersion enthalten sein, bevorzugt sind Mengen von 1 - 25 Gew.-% und insbesondere 5 - 20 Gew.-% bezogen auf die Wachsdispersion.

Als zusätzliche wachsartige Lipidkomponenten können auch C₁₄-C₄₀-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

### Wachse

Als weitere Lipidkomponente erfindungsgemäß verwendbar sind beispielsweise auch natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylen-glycolwachse. Pflanzliche Wachse sind erfindungsgemäß bevorzugt.

Die Wachskomponente kann ebenso gewählt werden aus der Gruppe der Ester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z.B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispielhaft sind hier zu erwähnen die C₁₆-C₄₀-Alkylstearate, C₂₀-C₄₀-Alkylstearate (z. B. Kesterwachs® K82H), C₂₀-C₄₀-Dialkylester von Dimersäuren, C₁₈-C₃₈-Alkylhydroxystearoylstearate oder C₂₀-C₄₀-Alkylerucate. Ferner sind C₃₀-C₅₀-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat vorteilhaft einsetzbar. Für Wipes für die Hautpflege ist u.a. Myristyllactat (Cegesoft® C17) besonders gut geeignet, da es ein gutes Bindevermögen zur Haut aufweist. Auch Siliconwachse sind gegebenenfalls vorteilhaft.

In einer bevorzugten Ausführungsform der Erfindung ist wenigstens eine weitere Lipidkomponente ausgewählt aus den C₁₂-C₂₄-Fettalkoholen, den Mono-, Di- oder Triestern aus Glycerin und C₁₂-C₂₄-Fettsäuren, den Mono- oder Diestern aus Ethylenglycol und C₁₂-C₂₄-Fettsäuren oder einem Gemisch dieser Substanzen enthalten. Erfindungsgemäß besonders bevorzugt ist ein C₁₂-C₂₄-Fettalkohol oder die Kombination wenigstens eines C₁₂-C₂₄-Fettalkohols mit Mono- oder Diestern aus Glycerin oder Ethylenglycol und C₁₂-C₂₄-Fettsäuren.

Als weitere Konsistenzgeber können ggf. geringe Mengen an Alkalimetall- und Erdalkalimetall- sowie Aluminiumsalze von C₁₂-C₂₄-Fettsäuren oder C₁₂-C₂₄-Hydroxyfettsäuren eingesetzt werden, wobei Calcium-, Magnesium-, Aluminium- und insbesondere Zinkstearat bevorzugt ist.

### Ölkörper

In einer weiteren bevorzugten Ausführungsform enthält die erfindungsgemäße Zusammensetzung wenigstens einen Ölkörper. Unter Ölkörpern sind erfindungsgemäß bei 20 °C flüssige, mit Wasser bei 25 °C nicht mischbare Stoffe oder Gemische von Stoffen zu verstehen. Hierzu gehören alle Ölkörper die nicht unter die in Anspruch 1 genannten Dialkyl(en)ether, Dialkyl(en)carbonate, Dicarbonsäure oder Hydroxyfettalkohole fallen, also z. B. bei 20 °C flüssige Glyceride, Kohlenwasserstoffe, Silikonöle, Esteröle oder beliebige Gemische davon. Die Ölkörper sind in den erfindungsgemäßen Zusammensetzungen üblicherweise in Mengen von weniger als 30 Gew.-%, bevorzugt in Mengen von 0,5 - 10 und insbesondere in Mengen von 0,5 - 5 Gew.-% bezogen auf die Wachsdispersion enthalten. Die Menge der eingearbeiteten Öle wird durch die Maßgabe limitiert, daß der Schmelzpunkt der Wachsphase oberhalb 25 °C liegen muß. Derartige Optimierungen gehören zu Routine-Optimierungen des Fachmanns.

Zu den als Ölkörper erfindungsgemäß einsetzbaren Glyceriden zählen bei 20 °C flüssige Fettsäureester des Glycerins, die natürlicher (tierischer und pflanzlicher) oder synthetischer Herkunft sein können. Man unterscheidet zwischen Mono-, Di- und Triglyceriden. Es handelt sich um bekannte Stoffe, die nach einschlägigen Verfahren der präparativen organischen Chemie hergestellt werden können. Synthetisch hergestellte Glyceride sind üblicherweise Mischungen von Mono-, Di- und Triglyceriden, die durch Umesterung der entsprechenden Triglyceride mit Glycerin oder durch gezielte Veresterung von Fettsäuren erhalten werden. Als Fettsäure sind erfindungsgemäß C₆-C₂₄-Fettsäuren, und unter diesen C₆-C₁₈-Fettsäuren, und insbesondere C₈-C₁₈-Fettsäuren bevorzugt geeignet. Die Fettsäuren können verzweigt oder unverzweigt, gesättigt oder ungesättigt sein. Erfindungsgemäß bevorzugt ist die Verwendung bei 20 °C flüssiger Glyceride pflanzlicher Herkunft, insbesondere von Cocoglyceriden, einer Mischung aus vorwiegend Di- und Triglyceriden mit C₈-C₁₈-Fettsäuren, die beispielsweise unter der Bezeichnung Myritol® 331 von der Cognis Deutschland GmbH vertrieben werden. Ebenso bevorzugt ist die Verwendung von Myritol® 312 (C₈/C₁₀-Triglyceride), Cegesoft® PS 17, Cegesoft® GPO; Cegesoft® PFO und Cegesoft® PS 6, die den Zusammensetzungen nach Applikation besonders gute pflegende Eigenschaften verleihen.

Als Ölkörper kommen auch bei 20° C flüssige Guerbetalkohole auf Basis von Fettalkoholen mit 6 bis 18, vorzugsweise 8 bis 10 Kohlenstoffatomen, wie beispielsweise Eutanol® G, in Frage. Auch flüssige Ester von linearen, gesättigten oder ungesättigten C₆-C₂₂-Fettsäuren mit linearen oder verzweigten, gesättigten oder ungesättigten C₆-C₂₂-Fettalkoholen bzw. Ester von verzweigten C₆-C₁₃-Carbonsäuren mit linearen oder verzweigten, gesättigten oder ungesättigten C₆-C₂₂-Fettalkoholen sind erfindungsgemäß als Ölkörper einsetzbar.

Unter den flüssigen Wachsestern seien expemplarisch folgende typische Vertreter genannt: Decyloleat (Cetiol® V), Cococaprylate/-caprat (Cetiol® SN), Hexyllaurat (Cetiol® A), Myristylisostearat, Myristyloleat, Cetylisostearat, Cetyloleat, Stearylisostearat, Isostearylmyristat, Isostearylpalmitat, Isostearylstearat, Isostearylisostearat, Isostearyloleat, Isostearyloleat, Oleylmyristat, Oleylisostearat, Oleyloleat, Oleylerucat (Cetiol®J 600), Behenylisostearat, Erucylisostearat, Erucyloleat. Daneben eignen sich auch Ester von linearen C₆-C₂₂-Fettsäuren mit verzweigten Alkoholen, insbesondere 2-Ethylhexanol (Cetiol® 868), Ester von verzweigten C₆-C₂₂-Fettsäuren mit linearen Alkoholen, Ester von C₁₈-C₃₈-Alkylhy-droxycarbonsäuren mit linearen oder verzweigten C₆-C₂₂-Fettalkoholen, Ester von linearen und/oder verzweigten Fettsäuren mit mehrwertigen Alkoholen (wie z. B. Propylenglycol, Dimerdiol oder Trimertriol) und/oder Guerbetalkoholen, sowie Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen (z. B. Dioctyl Malate).

Zu den erfindungsgemäß einsetzbaren Ölkörpern zählen auch bei 20 °C flüssige natürliche und synthetische, aliphatische und/oder naphthenische Kohlenwasserstoffe, wie z. B. Squalan, Squalen, Paraffinöle, Isohexadecan, Isoeicosan oder Polydecene sowie Dialkylcyclohexane (Cetiol® S).

Erfindungsgemäß sind als Ölkörper auch flüssige Siliconöle geeignet. Zu diesen zählen z. B. Dialkyl- und Alkylarylsiloxane, wie beispielsweise Cyclomethicone, Dimethylpolysiloxan und Methylphenylpolysiloxan, sowie deren alkoxylierte und quaternierte Analoga. Geeignete nicht-flüchtige Siliconöle sind z. B. Polyalkylsiloxane, Polyalkylarylsiloxane und Polyethersiloxan-Copolymere. Der Zusatz von Siliconverbindungen vermittelt ein besonders leichtes Hautgefühl.

In einer bevorzugten Ausführungsform enthält die erfindungsgemäße Wachsdispersion mit einer durchschnittlichen Partikelgröße (mittels Frauenhofer-Beugung) von 0,5 bis 100 nm
1- 50 Gew.-% einer Wachsphase, die
(a1) 0,1 - 30 Gew.-% wenigstens einer Öl- oder Wachskomponente ausgewählt aus C₁₄-C₃₀-Dialkyl(en)ethern, C₁₄-C₃₀-Dialkyl(en)carbonaten, C₉-C₃₄-Dicarbonsäuren oder C₁₂-C₃₀-Hydroxyfettalkoholen oder einem beliebigen Gemisch dieser Substanzen,
(a2) 0,1 - 10 Gew.-% wenigstens eines Öls,
(a3) 0,1 -10 Gew.-% wenigstens eines nicht-ionischen Emulgators,
(a4) 0,1 - 40 Gew.-% wenigstens einer weiteren wachsartigen Lipidkomponente bezogen auf die Gesamtzusammensetzung der Wachsdispersion enthält und
(b) 50 - 99 Gew.-% einer Wasserphase
bezogen auf die Gesamtzusammensetzung der Wachsdispersion umfasst.

### Polymere

In einer weiteren bevorzugten Ausführungsform enthält die erfindungsgemäße Zusammensetzung wenigstens ein Polymer, vorzugsweise in der Wasserphase. Diese trägt dazu bei, die Feinteiligkeit der Dispersion weiter zu verbessern. Die Polymere sind vorzugsweise in einer Menge von 0,01 - 5 Gew.-%, insbesondere 0,05 - 3 und besonders bevorzugt 0,1 - 2 Gew.-% bezogen auf die Wachsdispersion enthalten.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert. Butyl-aminoethylmethacrylat/2-Hydroxypropylmethacrylat-Copolymere, Polyvinylpyrrolidon, Vinylpyrro-lidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage. Erfindungsgemäß geeignet sind Polysaccharide, insbesondere Xanthan-Gum, Guar-Guar, Agar-Agar, Alginate und Tylosen, Carboxymethylcellulose und Hydroxyethylcellulose, Polyacrylate, (z.B. Carbopole® von Noveon oder Synthalene® von 3v/Sigma), Poly-acrylamide, Polyvinylalkohol und ferner höhermolekulare Polyethylenglycolmono- und - diester von Fettsäuren.

Geeignete **kationische Polymere** sind beispielsweise kationische Cellulosederivate, wie z. B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensations-produkte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z. B. Mirapol® A-15, Mirapol® AD-1, Mirapol® AZ-1 der Firma Miranol.

Erfindungsgemäß bevorzugt geeignet sind in Wasser lösliche oder mit Wasser quellbare Polymere, insbesondere nichtionische und anionische Polymere. Besonders bevorzugt geeignet sind Polymere, die ausgewählt sind aus der Gruppe der Polyacrylate, der Polysacharide, der Polyacrylamide oder einem beliebigen Gemisch dieser Substanzen.

### Wirkstoffe

Eine bevorzugte Ausführungsform der erfindungsgemäßen Zusammensetzung enthält zusätzlich wenigstens einen Wirkstoff. Unter Wirkstoffen werden erfindungsgemäß Stoffe verstanden, die zum Schutz der Haut und zur Stärkung der Hautbarriere beitragen, die reizlindernd, antimikrobiell oder hautbefeuchtend wirken. Erfindungsgemäß bevorzugt sind Wirkstoffe, die zur Linderung entzündlicher Hautprozesse oder geröteter, wunder Haut dienen, zu denen beispielsweise auch Zinkverbindungen oder Schwefel zählen. Der Wirkstoff ist - je nach Art - üblicherweise in einer Menge von 0,01 - 10 Gew.-%, vorzugsweise 0,1 - 7 Gew.-% und insbesondere 1 - 5 Gew.-% bezogen auf die Wachsdispersion enthalten. Bevorzugt sind öllösliche Wirkstoffe, obgleich sich durch Zugabe von Emulgatoren und/oder Solubilisatoren auch begrenzte Mengen wasserlöslicher Wirkstoffe einarbeiten lassen. Die Wirkstoffe können auch in beliebiger Kombination eingesetzt werden.

Geeignet sind z. B. auch Pflanzenextrakte, die häufig eine synergistisch wirkende Kombination wundheilender/reizlindernder Stoffe enthalten. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Kamille, Aloe Vera, Hamamelis, Lindenblüten, Roßkastanie, Grünem Tee, Eichenrinde, Brennessel, Hopfen, Klettenwurzel, Schachtelhalm, Weißdorn, Mandel, Fichtennadel, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel geeignet.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

### Antimikrobielle/biogene Wirkstoffe

Typische Beispiele für **keimhemmende Mittel** sind Konservierungsmittel mit spezifischer Wirkung gegen gram-positive Bakterien wie etwa 2,4,4'-Trichlor-2'-hydroxydiphenylether, Chlorhexidin (1,6-Di-(4-chlorphenyl-biguanido)hexan) oder TCC (3,4,4'-Trichlorcarbanilid). Auch zahlreiche Riechstoffe und etherische Öle weisen antimikrobielle Eigenschaften auf. Typische Beispiele sind die Wirkstoffe Eugenol, Menthol und Thymol in Nelken-, Minz- und Thymianöl. Ein interessantes natürliches Deomittel ist der Terpenalkohol Farnesol (3,7,11-Trimethyl-2,6,10-dodecatrien-1-ol), der im Lindenblütenöl vorhanden ist und einen Maiglöckchengeruch hat. Auch Glycerinmonolaurat, Glycerinstearat, Glycerinoleat sowie Glycerindioleat haben sich als keimhemmend erwiesen und sind wegen ihrer außerordentlichen Milde und Unbedenklichkeit besonders im Bereich der Baby-Hygiene und -Pflege vorteilhaft einsetzbar. Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, α-Hydroxycarbonsäuren, Aminosäuren, Ceramide, Pseudoceramide, essentielle Öle, Pflanzenextrakte und Vitaminkomplexe zu verstehen. Erfindungsgemäß bevorzugt als Wirkstoffe sind öllösliche Vitamine und Vitaminvorstufen. Ganz besonders bevorzugt sind Tocopherol (Vitamin-E) und Tocopherol-Derivate.

Üblicherweise liegt der Anteil der keimhemmenden Mittel bei etwa 0,1 bis 2 Gew.-% - bezogen auf die Wachsdispersion. Die Glycerinester sind in höheren Mengen einsetzbar (vide supra).

### Feuchthaltemittel/Hautbefeuchtungsmittel

In einer weiteren bevorzugten Ausführungsform enthält die erfindungsgemäße Zusammensetzung zusätzlich wenigstens ein Feuchthaltemittel. Dieses dient zur Verbesserung der sensorischen Eigenschaften der Zusammensetzung sowie zur Feuchtigkeitsregulierung der Haut. Es kann außerdem dazu beitragen, das Eindringvermögen der Zusammensetzung auf den Wipes zu verbessern. Feuchthaltemittel sind üblicherweise in einer Menge von 0,1 - 10 Gew.-%, vorzugsweise 0,5 - 10 Gew.-%, und insbesondere 0,5 -5 Gew.-% bezogen auf die Wachsdispersion enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin und Triglycerin.

### Verfahren

Gegenstand der vorliegenden Erfindung ist auch ein Verfahren zur Herstellung einer Wachsdispersion mit einer durchschnittlichen Partikelgröße (mittels Frauenhofer-Beugung) von 0,5 bis 100 µm, dadurch gekennzeichnet, daß man eine Voremulsion der Wachsphase mit einer Wasserphase herstellt und diese heiße Voremulsion, die eine Temperatur oberhalb des Schmelzbereiches der Wachse aufweist, in eine kalte polymerhaltige Wasserphase, die eine Temperatur von 1 - 30 °C aufweist, einbringt. Vorzugsweise ist die Temperatur der Wasserphase kleiner oder gleich 25 °C, besonders bevorzugt ist eine Temperatur von 5 - 25 °C. Die Temperatur dieser kalten Wasserphase sollte während Zugabe der "wärmeren" Voremulsion nicht den Schmelzbereich der verwendeten Wachse erreichen, und ist daher entsprechend zu kühlen. Die Wachskomponenten können unter allen vorher genannten Wachskomponenten ausgewählt werden.

Das Polymer ist vorzugsweise wasserlöslich oder wasserquellbar. Die Polymere erlauben es, sehr fein-partikuläre und stabile Wachsdispersionen zu erhalten, die sich auch bei langer Lagerzeit nicht trennen. Vorzugsweise enthält auch die Voremulsion ein Polymer. Geeignete Polymere wurden bereits beschrieben (vide supra). In einer bevorzugten Ausführungsform des Verfahrens ist das Polymer ausgewählt aus der Gruppe der Polyacrylate, der Polysaccharide, der Polyacrylamide oder einem beliebigen Gemisch dieser Substanzen.

Das Einbringen der "heißen" Voremulsion in die "kalte" Wasserphase kann mit Hilfe der üblichen Methoden erfolgen, die dem Fachmann hinlänglich bekannt sind. Zur Erzielung besonders feinteiliger Tröpfchen wird die Voremulsion vorzugsweise wenigstens einmal homogenisiert, bevor sie in die Wasserphase eingebracht wird. Die Homogenisierung kann beispielsweise als Hochdruckhomogenisierung erfolgen. In einer weiteren bevorzugten Ausführungsform wird die Voremulsion vor dem Einbringen in die Wasserphase über einen Wärmeaustauscher abgekühlt, vorzugsweise auf Temperaturen unterhalb von 50° C. Vorzugsweise wird die Voremulsion unter Druck durch eine Düse in die Wasserphase eingesprüht. Die Einstellung der exakten Druckbedingungen ist dabei apparateabhängig.

In einer weiteren bevorzugten Ausführungsform des Verfahrens enthält die Wachsphase wenigstens eine Öl- oder Wachskomponente ausgewählt aus den Dialkyl(en)ethern, Dialkyl(en)carbonaten, Dicarbonsäuren oder Hydroxyfettalkoholen oder einem beliebigen Gemisch dieser Substanzen und wenigstens einen Emulgator. Auch diese Substanzen wurden bereits eingehend offenbart. Vorzugsweise liegt der Schmelzpunkt der Wachsphase oberhalb 25 °C.

Erfindungsgemäß bevorzugt ist es, die Einsatzmengen so zu wählen, daß gemäß dem erfindungsgemäßen Verfahren Wachsdispersionen resultieren, die (a) 10 - 75 Gew.-% einer Wachsphase und (b) 25 - 90 Gew.% einer Wasserphase bezogen auf die Gesamtzusammensetzung umfassen.

### Weitere Hilfs- und Zusatzstoffe

Die erfindungsgemäßen Zusammensetzungen können je nach Art und Zweck der Applikation eine Reihe weiterer Hilfs- und Zusatzstoffe enthalten, wie beispielsweise Überfettungsmittel, weitere Verdickungsmittel, Puder, biogene Wirkstoffe, Deowirkstoffe, Filmbildner, UV-Lichtschutzfaktoren, Antioxidantien, Hydrotrope, Konservierungsmittel, Insektenrepellentien, Selbstbräuner, Solubilisatoren, Parfümöle, Farbstoffe, und dergleichen.

Als UV-Lichtschutzfaktoren (z. B. bei Sunscreen-Wipes) werden vorzugsweise Derivate des Benzophenons eingesetzt, z. B. 2-Hydroxy-4-methoxybenzophenon, 2-Hydroxy-4-methoxy-4'-methylbenzophenon und/oder 2,2'-Dihydroxy-4-methoxybenzophenon.

Bevorzugte **Antioxidantien** sind Carotinoide, Carotine (z.B. α-Carotin, β-Carotin, Lycopin) und deren Derivate, α-Hydroxysäuren (z.B. Citronensäure, Milchsäure, Apfelsäure), EDTA, Vitamin C und Derivate (z.B. Ascorbylpalmitat, Mg-Ascorbylphosphat, Ascorbylacetat), Tocopherole und Derivate (z.B. Vitamin-E-acetat), Butylhydroxytoluol sowie Butylhydroxyanisol.

### Beispiele

### Herstellvorschrift

### Herstellprozeß der Wachsdispersion in 1000 kg Anlage:

In einen heizbaren Rührkessel 1 (3m³) wurden Distearylcarbonat (20 kg), Distearylether (20 kg), Lorol® C12 (50 kg), Lanette® 14 (20 kg), Lanette® 16 (20 kg), Lanette® 18 (10 kg), Paraffinöl perl. DAB (20 kg), ein Anteil des Emulgators Eumulgin® B2 (3,0 kg), Dehymuls® PGPH (1,0 kg), ein Anteil des Polymers Sepigel® 305 (1,0 kg) sowie ein Anteil des VE Wassers (430,0 kg) gegeben. Dieser Behälter wurde auf Temperaturen oberhalb der Schmelztemperatur der eingesetzten Wachse (in diesem Fall ca. 80-90°C) erwärmt und solange kräftig gerührt (Propellerrührer bei ca. 200 upm), bis eine homogen verteilte Voremulsion entstanden war. Diese Voremulsion wurde nun aus dem Rührkessel 1, über eine Kreiselradpumpe, die einen ungefähren Vordruck von 2 bar erzeugt, durch den Supraton®-Homogenisator geleitet. Es wurde zunächst ca. 30 Minuten in einem Kreislauf gefahren, d.h. die Voremulsion wurde nach der Kreiselradpumpe über den Supraton®-Homogenisator (Hersteller: Supraton) und dann zurück in den Rührkessel 1 gepumpt. Nach dem 30-minütigen Umpumpen im Kreislauf, wurde die Voremulsion dann vom Supraton® nicht mehr zurück in den Rührkessel 1, sondern über einen Plattenwärmeaustauscher (W. Schmidt GmbH) gepumpt. Der Plattenwärmeaustauscher wurde über einen Kaltwasserkreislauf (ca. 7°C) gekühlt. Die homogenisierte Voremulsion wurde auf diese Weise über den Plattenwärmeaustauscher auf ca. 50°C abgekühlt und anschließend, bei einem ungefähren Druck von 2 bar, in den Rührkessel 2 eingetragen. Der Eintrag erfolgte über eine Düse, die sich unterhalb des Flüssigkeitsspiegels befand, d.h. ohne Lufteintrag zu ermöglichen.

In Rührkessel 2 (1m³), d.h. dem Auffangkessel, wurde der restliche Anteil VE Wasser (396,0 kg), die restlichen Anteile des gelösten nichtionischen Emulgators Eumulgin® B2 (1,0 kg) sowie des Polymers Sepigel® 305 (3,0 kg) vorgelegt und auf etwa 5°C gekühlt. Die Zugabe der Voremulsion muß unter effizienter Rührung (Propellerrührer bei ca. 120 upm) und unter externer Mantelkühlung des Rührkessels 2 erfolgen, so dass beim Einbringen der Voremulsion eine Temperatur von unterhalb 25°C gewährleistet wurde. Im Anschluss wurden Phenoxyethanol (5,0 kg) unter ständigem Rühren zur Konservierung hinzugegeben.

Die Viskosität dieser Wachsdispersion, gemessen mit Brookfield RVF, Spindel 5, 10 rpm betrug 30.000 mPa·s bei 23° C. Die Teilchengrößenmessung erfolgte mittels Fraunhofer-Beugung (Mastersizer 2000, Malvern Instrumentes Ltd.) und ergab eine Teilchengrößenverteilung d(0.5) von 10 Mikrometern und d(0.9) von 30 Mikrometer, d.h. 50% des dispergierten Materials waren kleiner als 10 Mikrometer und 90% kleiner als 30 Mikrometer.

Zur Prüfung der anwendungstechnischen Eigenschaften wurde die Stabilität und Sensorik der erfindungsgemäßen Zusammensetzung überprüft. Handelsübliche Wipes (Substrat) mit einem Gewicht von 55 g/m² wurden mit den erfindungsgemäßen Wachsdispersionen in einer Menge von jeweils 165 g/m² beschichtet. Die erfindungsgemäßen Zusammensetzungen sind vorteilhafter als Zusammensetzungen des Standes der Technik applizierbar und sind bezüglich Sensorik und Lagerstabilität den herkömmlichen Zusammensetzungen des Standes der Technik überlegen.

Die Mengenangaben in nachfolgenden Beispielen beziehen sich, soweit nicht anders angegeben, auf Gew.-% der handelsüblichen Substanzen in der Gesamtzusammensetzung (Wachsdispersion). Die Beispiele 1 bis 5 sind erfindungsgemäße Formulierungen, V1 ist ein Vergleichsbeispiel.

**Tabelle 1 (Wachsdispersionen):**

| **Inhaltsstoffe** | **Bsp. 1 Gew %** | **Bsp. 2 Gew %** | **Bsp. 3 Gew %** | **Bsp. 4 Gew %** | **Bsp. 5 Gew %** | **V 1 Gew %** |
|---|---|---|---|---|---|---|
| Distearyl Carbonate | 3,0 | - | 2,5 | - | 1,0 | - |
| Distearyl Ether | - | 2,0 | 2,5 | - | - | - |
| Dibehenyl Ether | - | 1,0 | - | - | - | - |
| Azelainsäure | 2,0 | - | - | 1,0 | 1,0 | - |
| 12-Hydroxystearylalkohol | - | 4,0 | - | 2,5 | - | - |
| Lorol® C12 | 3,0 | 2,0 | 3,0 | 4,0 | 1,0 | 2,0 |
| Lanette® 14 | 2,0 | 2,0 | 3,0 | 2,0 | 1,0 | 4,0 |
| Lanette® 16 | 2,0 | 3,0 | 4,0 | 3,0 | 0,5 | 3,0 |
| Lanette® 18 | 0,5 | 1,0 | 1,0 | - | 0,5 | 2,0 |
| Lanette® O | - | - | - | 2,0 | - | - |
| Cutina® MD | - | 2,0 | - | - | 1,0 | - |
| Cutina® AGS | - | - | 2,0 | - | - | - |
| Novata® B | - | - | 0,5 | 5,0 | 3,0 | - |
| Cegesoft® HF 52 | - | - | - | 1,0 | 2,0 | - |
| Cegesoft® SH | - | - | - | - | 5,0 | - |
| Emulgade® PL 68/50 | 0,5 | - | - | 1,0 | 2,0 | 0,5 |
| Eumulgin® VL 75 | - | - | 1,0 | - | - | - |
| Eumulgin® B1 | - | 2,0 | 0,2 | 0,5 | - | - |
| Eumulgin® B2 | 0,5 | - | - | - | - | - |
| Lanette® E | - | - | - | 0,1 | - | - |
| Dehymuls® PGPH | - | 0,2 | 0,2 | - | - | - |
| Cetiol® 868 | 0,5 | - | 1,0 | - | 1,0 | 0,5 |
| Cetiol® OE | - | 1,0 | 0,5 | - | - | - |
| Mineralöl (Klearol) | - | 0,5 | - | - | - | - |
| Cegesoft® PS 6 | - | - | - | 1,0 | - | - |
| Cetiol® CC | 1,0 | - | - | 1,0 | - | 1,0 |
| Myritol® 331 | - | 0,2 | 0,5 | 0,5 | - | - |
| Natrosol® 250 HR | 0,2 | - | - | - | - | - |
| Hispagel® 200 | - | 3,0 | 1,0 | - | - | - |
| Jaguar® HP-105 | - | - | - | 0,4 | - | - |
| Cosmedia® SP | - | 0,3 | - | - | 0,3 | - |
| Sepigel® 305 | 0,2 | - | 0,4 | - | - | - |
| Panthenol | 1,0 | - | - | - | - | 1,0 |
| Butylene Glycol | - | - | - | 1,0 | - | - |
| Copherol® F1300 | - | - | - | 0,5 | - | - |
| Neo Heliopan, Type BB | - | - | - | 0,5 | - | - |
| Glycerin | - | - | 1,0 | - | 2,0 | - |
| Bisabolol | - | 0,5 | - | - | - | - |
| Hibiscin® HP LS 9198) | - | - | 1,0 | - | - | - |
| | | | | | | |
| Konservierungsmittel | qs | qs | qs | Qs | qs | qs |
| Wasser, demineralisiert | Ad 100 | Ad 100 | Ad 100 | Ad 100 | Ad 100 | Ad 100 |
| ***Feinteiligkeit* / *Stabilität der Dispersion*** | + | + | + | + | + | - |

### Anhang

1) Cegesoft® HF 52
   INCI: Hydrogenated Vegetable Oil
   Hersteller: Cognis Deutschland GmbH & Co. KG
2) Cegesoft® PS 6
   INCI: Vegetable Oil
   Hersteller: Cognis Deutschland GmbH & Co. KG
3) Cegesoft® SH
   INCI: Shorea Stenoptera
   Hersteller: Cognis Deutschland GmbH & Co. KG
4) Cetiol® CC
   INCI: Dicaprylyl Carbonate
   Hersteller: Cognis Deutschland GmbH & Co. KG
5) Cetiol® OE
   INCI: Dicaprylyl Ether
   Hersteller: Cognis Deutschland GmbH & Co. KG
6) Cetiol® 868
   INCI: Octyl Stearate
   Hersteller: Cognis Deutschland GmbH & Co. KG
7) Copherol® F 1300
   INCI: Tocopherol
   Hersteller: Cognis Deutschland GmbH & Co. KG
8) Cosmedia® SP
   INCI: Sodium Polyacrylate
   Hersteller: Cognis Deutschland GmbH & Co. KG
9) Cutina® AGS
   INCI : Glycol Distearate
   Hersteller: Cognis Deutschland GmbH & Co. KG
10) Cutina® MD
   INCI: Glyceryl Stearate
   Hersteller: Cognis Deutschland GmbH & Co. KG
11) Dehymuls® PGPH
   INCI: Polyglyceryl-2 Dipolyhydroxystearate
   Hersteller: Cognis Deutschland GmbH & Co. KG
12) Emulgade® PL 68/50
   INCI: Cetearyl Glucoside, Cetearyl Alcohol
   Hersteller: Cognis Deutschland GmbH & Co. KG
13) Eumulgin ® B1
   INCI: Ceteareth-12
   Hersteller: Cognis Deutschland GmbH & Co. KG
14) Eumulgin® B2
   INCI: Ceteareth-20
   Hersteller: Cognis Deutschland GmbH & Co. KG
15) Eumlugin® VL 75
   INCI: Lauryl Glucoside, Polyglyceryl-2 Dipolyhydroxystearate, Glycerin, Aqua (Water); ca. 75 % Aktivsubstanz in Wasser
   Hersteller: Cognis Deutschland GmbH & Co. KG
16) Hibiscin® HP LS 9198
   INCI: Water and Hibiscus esculentus seed extract
   Hersteller: Laboratoires Sérobiologiques
17) Hispagel® 200
   INCI: Glycerin, Glyceryl Polyacrylate
   Hersteller: Cognis Iberia
18) Jaguar HP-105
   INCI: Hydroxypropyl Guar
   Hersteller: Rhodia
19) Lanette® 14
   INCI: Myristyl Alcohol
   Hersteller: Cognis Deutschland GmbH & Co. KG
20) Lanette® 16
   INCI: Cetyl Alcohol
   Hersteller: Cognis Deutschland GmbH & Co. KG
21) Lanette® 18
   INCI: Stearyl Alcohol
   Hersteller: Cognis Deutschland GmbH & Co. KG
22) Lanette E
   INCI: Sodium Cetearyl Sulfate
   Hersteller: Cognis Deutschland GmbH & Co. KG
23) Lanette® O
   INCI: Cetearyl Alcohol
   Hersteller: Cognis Deutschland GmbH & Co. KG
24) Lorol® C12
   INCI: Lauryl Alcohol
   Hersteller: Cognis Deutschland GmbH & Co. KG
25) Myritol® 331
   INCI: Cocoglycerides
   Hersteller: Cognis Deutschland GmbH & Co. KG
26) Natrosol® 250 HR
   INCI: Hydroxyethylcellulose
   Hersteller: Hercules Inc.
27) Neo Heliopan, Type BB
   INCI: Benzophenone-3
   Hersteller: Haarman & Reimer GmbH
28) Novata® B
   INCI: Cocoglycerides
   Hersteller: Cognis Deutschland GmbH & Co. KG
29) Sepigel® 305
   INCI: Polyacrylamide, C13-C14 Isoparaffin, Laureth-7
   Hersteller: SEPPIC

## Patentansprüche

1. Wachsdispersion mit einer durchschnittlichen Partikelgröße (mittels Fraunhofer-Beugung) von 0,5 bis 100 µm umfassend
(a) 10-75 Gew.-% bezogen auf die Wachsdispersion einer Wachsphase, die im Bereich oberhalb 25°C und bis zu 50°C schmilzt, die wenigstens eine Öl- oder Wachskomponente ausgewählt aus den Dialkyl(en)ethern, Dialkyl(en)carbonaten, Dicarbonsäuren oder Hydroxyfettalkoholen oder einem beliebigen Gemisch dieser Substanzen und wenigstens einen Emulgator enthält und
(b) eine Wasserphase.

2. Wachsdispersion gemäß Anspruch 1, **dadurch gekennzeichnet, dass** sie 0,1 - 10 Gew.-% Emulgator(en) bezogen auf die Gesamtzusammensetzung umfaßt.

3. Wachsdispersion gemäß wenigstens einem der Ansprüche 1 bis 2, **dadurch gekennzeichnet, dass** die Wachsphase wenigstens einen Emulgator ausgewählt aus der Gruppe der nicht-ionischen Emulgatoren enthält.

4. Wachsdispersion gemäß wenigstens einem der Ansprüche 1 bis 3, **dadurch gekennzeichnet, dass** die Wachsphase im Bereich von 35 - 50°C schmilzt.

5. Wachsdispersion gemäß wenigstens einem der Ansprüche 1 bis 4, **dadurch gekennzeichnet, dass** die Wachsdispersion Partikel mit einer durchschnittlichen Teilchengröße von 1 - 50 µm, vorzugsweise 5 - 30 µm aufweist.

6. Wachsdispersion gemäß wenigstens einem der Ansprüche 1 bis 5, **dadurch gekennzeichnet, dass** die Wachsphase wenigstens eine weitere wachsartige Lipidkomponente enthält.

7. Wachsdispersion gemäß Anspruch 6, **dadurch gekennzeichnet, dass** die weitere wachsartige Lipidkomponente ausgewählt ist aus den C₁₂-C₂₄-Fettalkoholen, den Mono-, Di- oder Triestern aus Glycerin und C₁₂-C₂₄-Fettsäuren, den Mono- oder Diestern aus Ethylenglycol und C₁₂-C₂₄-Fettsäuren oder beliebigen Gemischen davon.

8. Wachsdispersion gemäß wenigstens einem der Ansprüche 1 bis 7, **dadurch gekennzeichnet, dass** die Wachsphase zusätzlich wenigstens einen weiteren Ölkörper enthält.

9. Wachsdispersion mit einer durchschnittlichen Partikelgröße (mittels Fraunhofer-Beugung) von 0,5 bis 100 nm, **dadurch gekennzeichnet, dass** sie 1 - 50 Gew.-% einer Wachsphase, die
(a1) 0,1 - 30 Gew.-% wenigstens einer Öl- oder Wachskomponente ausgewählt aus C₁₄-C₃₀-Dialkyl(en)ethern, C₁₄-C₃₀-Dialkyl(en)carbonaten, C₉-C₃₄-Dicarbonsäuren oder C₁₂-C₃₀-Hydroxyfettalkoholen oder einem beliebigen Gemisch dieser Substanzen,
(a2) 0,1 - 10 Gew.-% wenigstens eines Öls,
(a3) 0,1 - 10 Gew.-% wenigstens eines nicht-ionischen Emulgators,
(a4) 0,1 - 40 Gew.-% wenigsten einer weiteren wachsartigen Lipidkomponente
bezogen auf die Gesamtzusammensetzung der Wachsdispersion enthält und
(b) 50 - 99 Gew.-% einer Wasserphase
bezogen auf die Gesamtzusammensetzung umfasst.

10. Wachsdispersion gemäß wenigstens einem der Ansprüche 1 bis 9, **dadurch gekennzeichnet, dass** sie zusätzlich wenigstens ein Polymer, vorzugsweise in einer Menge von 0,01 - 5,0 Gew.-% bezogen auf die Wachsdispersion, enthält.

11. Wachsdispersion gemäß wenigstens einem der Ansprüche 1 bis 10, **dadurch gekennzeichnet, dass** das Polymer ausgewählt ist aus der Gruppe der Polyacrylate, der Polysaccharide, der Polyacrylamide oder einem beliebigen Gemisch dieser Polymere.

12. Wachsdispersion gemäß einem der Ansprüche 1 bis 11, **dadurch gekennzeichnet, dass** die Wachsphase zusätzlich wenigstens einen Wirkstoff enthält.

13. Wachsdispersion gemäß wenigstens einem der Ansprüche 1 bis 12, **dadurch gekennzeichnet, dass** sie zusätzlich wenigstens ein Feuchthaltemittel enthält.

14. Verfahren zur Herstellung einer Wachsdispersion mit einer durchschnittlichen Partikelgröße (mittels Fraunhofer-Beugung) von 0,5 bis 100 µm, **dadurch gekennzeichnet, dass** man eine Voremulsion der Wachsphase mit einer Wasserphase herstellt, und diese Voremulsion, die eine Temperatur oberhalb des Schmelzbereiches der Wachse aufweist, in eine polymerhaltige Wasserphase, die eine Temperatur von 1 - 30 °C aufweist, unter Druck einbringt.

15. Verfahren gemäß Anspruch 14, **dadurch gekennzeichnet, dass** die Voremulsion in einem zwischengeschalteten Schritt wenigstens einmal homogenisiert wird, bevor sie in die Wasserphase eingebracht wird.

16. Verfahren gemäß wenigstens einem der Ansprüche 14 bis 15, **dadurch gekennzeichnet, dass** die Voremulsion vor dem Einbringen in die Wasserphase über einen Wärmeaustauscher abgekühlt wird.

17. Verfahren gemäß wenigstens einem der Ansprüche 14 bis 16, **dadurch gekennzeichnet, dass** die Voremulsion ebenfalls ein Polymer enthält.

18. Verfahren gemäß wenigstens einem der Ansprüche 14 bis 17, **dadurch gekennzeichnet, dass** das Polymer ausgewählt ist aus der Gruppe der Polyacrylate, der Polysaccharide, der Polyacrylamide oder einem beliebigen Gemisch dieser Substanzen.

19. Verfahren zur Herstellung einer Wachsdispersion gemäß wenigstens einem der Ansprüche 14 bis 18, **dadurch gekennzeichnet, dass** die Voremulsion unter Druck durch eine Düse in die Wasserphase eingesprüht wird.

20. Verfahren gemäß wenigstens einem der Ansprüche 14 bis 19, **dadurch gekennzeichnet, dass** die Wachsphase wenigstens eine Öl- oder Wachskomponente ausgewählt aus den Dialkyl(en)ethern, Dialkyl(en)carbonaten, Dicarbonsäuren oder Hydroxyfettalkoholen oder einem beliebigen Gemisch dieser Substanzen und wenigstens einen Emulgator enthält.

21. Verfahren gemäß wenigstens einem der Ansprüche 14 bis 20, **dadurch gekennzeichnet, dass** die Wachsphase eine Schmelzpunkt oberhalb 25 °C aufweist.

22. Verfahren gemäß wenigstens einem der Ansprüche 14 bis 21, **dadurch gekennzeichnet, dass** die resultierende Wachsdispersion
(a) 10 - 75 Gew.-% einer Wachsphase und
(b) 25 - 90 Gew.-% einer Wasserphase
bezogen auf die Gesamtzusammensetzung umfaßt.

23. Verwendung einer Wachsdispersion gemäß einem der Ansprüche 1 bis 13 als Körperpflegemittel oder zur Herstellung von Körperpflegemittel.

## Claims

1. Wax dispersion having an average particle size (by means of Fraunhofer diffraction) of from 0.5 to 100 µm, comprising
(a) from 10 to 75% by weight, based on the wax dispersion, of a wax phase which melts in the range above 25°C and up to 50°C, which comprises at least one oil or wax component selected from the dialkyl(ene) ethers, dialkyl(ene) carbonates, dicarboxylic acids or hydroxy fatty alcohols or any desired mixture of these substances, and at least one emulsifier, and
(b) a water phase.

2. Wax dispersion according to Claim 1, **characterized in that** it comprises from 0.1 to 10% by weight emulsifier(s), based on the total composition.

3. Wax dispersion according to at least one of Claims 1 to 2, **characterized in that** the wax phase comprises at least one emulsifier selected from the group of the non-ionic emulsifiers.

4. Wax dispersion according to at least one of Claims 1 to 3, **characterized in that** the wax phase melts in the range of from 35 to 50°C.

5. Wax dispersion according to at least one of Claims 1 to 4, **characterized in that** the wax dispersion comprises particles having an average particle size of from 1 to 50 µm, preferably from 5 to 30 µm.

6. Wax dispersion according to at least one of Claims 1 to 5, **characterized in that** the wax phase comprises at least one further wax-like lipid component.

7. Wax dispersion according to Claim 6, **characterized in that** the further wax-like lipid component is selected from the C₁₂-C₂₄-fatty alcohols, the mono-, di- or tri-esters of glycerol and C₁₂-C₂₄-fatty acids, the mono- or di-esters of ethylene glycol and C₁₂-C₂₄-fatty acids or any desired mixtures thereof.

8. Wax dispersion according to at least one of Claims 1 to 7, **characterized in that** the wax phase additionally comprises at least one further oily substance.

9. Wax dispersion having an average particle size (by means of Fraunhofer diffraction) of from 0.5 to 100 nm, **characterized in that** it comprises from 1 to 50% by weight of a wax phase which comprises
(a1) from 0.1 to 30% by weight of at least one oil or wax component selected from C₁₄-C₃₀-dialkyl (ene) ethers, C₁₄-C₃₀-dialkyl (ene) carbonates, C₉-C₃₄-dicarboxylic acids or C₁₂-C₃₀-hydroxy fatty alcohols or any desired mixture of these substances,
(a2) from 0.1 to 10% by weight of at least one oil,
(a3) from 0.1 to 10% by weight of at least one non-ionic emulsifier,
(a4) from 0.1 to 40% by weight of at least one further wax-like lipid component,
based on the total composition of the wax dispersion, and
(b) from 50 to 99% by weight of a water phase,
based on the total composition.

10. Wax dispersion according to at least one of Claims 1 to 9, **characterized in that** it additionally comprises at least one polymer, preferably in an amount of from 0.01 to 5.0% by weight, based on the wax dispersion.

11. Wax dispersion according to at least one of Claims 1 to 10, **characterized in that** the polymer is selected from the group of the polyacrylates, polysaccharides, polyacrylamides or any desired mixture of these polymers.

12. Wax dispersion according to one of Claims 1 to 11, **characterized in that** the wax phase additionally comprises at least one active ingredient.

13. Wax dispersion according to at least one of Claims 1 to 12, **characterized in that** it additionally comprises at least one humectant.

14. Process for the production of a wax dispersion having an average particle size (by means of Fraunhofer diffraction) of from 0.5 to 100 µm, **characterized in that** a pre-emulsion of the wax phase having a water phase is prepared, and the pre-emulsion, which has a temperature above the melting range of the wax, is introduced under pressure into a polymer-containing water phase which has a temperature of from 1 to 30°C.

15. Process according to Claim 14, **characterized in that** the pre-emulsion is homogenized at least once in an intermediate step before it is introduced into the water phase.

16. Process according to at least one of Claims 14 to 15, **characterized in that** the pre-emulsion is cooled *via* a heat exchanger before it is introduced into the water phase.

17. Process according to at least one of Claims 14 to 16, **characterized in that** the pre-emulsion likewise comprises a polymer.

18. Process according to at least one of Claims 14 to 17, **characterized in that** the polymer is selected from the group of the polyacrylates, polysaccharides, polyacrylamides or any desired mixture of these substances.

19. Process for the production of a wax dispersion according to at least one of Claims 14 to 18, **characterized in that** the pre-emulsion is sprayed into the water phase under pressure through a nozzle.

20. Process according to at least one of Claims 14 to 19, **characterized in that** the wax phase comprises at least one oil or wax component selected from the dialkyl(ene) ethers, dialkyl(ene) carbonates, dicarboxylic acids or hydroxy fatty alcohols or any desired mixture of these substances, and at least one emulsifier.

21. Process according to at least one of Claims 14 to 20, **characterized in that** the wax phase has a melting point above 25°C.

22. Process according to at least one of Claims 14 to 21, **characterized in that** the resulting wax dispersion comprises
(a) from 10 to 75% by weight of a wax phase and
(b) from 25 to 90% by weight of a water phase,
based on the total composition.

23. Use of a wax dispersion according to one of Claims 1 to 13 as a body care product or for the production of body care product.

## Revendications

1. Dispersion de cire ayant une taille de particule moyenne (par diffraction de Fraunhofer) de 0,5 à 100 µm, comprenant :
(a) 10 à 75 % en poids, par rapport à la dispersion de cire, d'une phase cireuse, qui fond dans la plage supérieure à 25 °C et jusqu'à 50 °C, qui contient au moins un composant huileux ou cireux choisi parmi les éthers de dialkyl(ène), les carbonates de dialkyl(ène), les acides dicarboxyliques ou les alcools hydroxy-gras ou un mélange quelconque de ces substances et au moins un émulsifiant, et
(b) une phase aqueuse.

2. Dispersion de cire selon la revendication 1, **caractérisée en ce qu'**elle comprend 0,1 à 10 % en poids d'émulsifiant(s), par rapport à la composition totale.

3. Dispersion de cire selon au moins l'une quelconque des revendications 1 à 2, **caractérisée en ce que** la phase cireuse contient au moins un émulsifiant choisi dans le groupe des émulsifiants non ioniques.

4. Dispersion de cire selon au moins l'une quelconque des revendications 1 à 3, **caractérisée en ce que** la phase cireuse fond dans la plage allant de 35 à 50 °C.

5. Dispersion de cire selon au moins l'une quelconque des revendications 1 à 4, **caractérisée en ce que** la dispersion de cire comprend des particules ayant une taille de particule moyenne de 1 à 50 µm, de préférence de 5 à 30 µm.

6. Dispersion de cire selon au moins l'une quelconque des revendications 1 à 5, **caractérisée en ce que** la phase cireuse contient au moins un autre composant lipidique de type cire.

7. Dispersion de cire selon la revendication 6, **caractérisée en ce que** l'autre composant lipidique de type cire est choisi parmi les alcools gras en C₁₂-C₂₄, les mono-, di- ou triesters de glycérine et d'acides gras en C₁₂-C₂₄, les mono- ou diesters d'éthylène glycol et d'acides gras en C₁₂-C₂₄ ou leurs mélanges quelconques.

8. Dispersion de cire selon au moins l'une quelconque des revendications 1 à 7, **caractérisée en ce que** la phase cireuse contient en outre au moins un corps huileux supplémentaire.

9. Dispersion de cire ayant une taille de particule moyenne (par diffraction de Fraunhofer) de 0,5 à 100 nm, **caractérisée en ce qu'**elle comprend 1 à 50 % en poids d'une phase cireuse qui contient
(a1) 0,1 à 30 % en poids d'au moins un composant huileux ou cireux choisi parmi les éthers de dialkyl(ène) en C₁₄-C₃₀, les carbonates de dialkyl (ène) en C₁₄-C₃₀, les acides dicarboxyliques en C₉-C₃₄ ou les alcools hydroxy-gras en C₁₂-C₃₀ ou un mélange quelconque de ces substances,
(a2) 0,1 à 10 % en poids d'au moins une huile,
(a3) 0,1 à 10 % en poids d'au moins un émulsifiant non ionique,
(a4) 0,1 à 40 % en poids d'au moins un autre composant lipidique de type cire, par rapport à la composition totale de la dispersion de cire, et
(b) 50 à 99 % en poids d'une phase aqueuse,
par rapport à la composition totale.

10. Dispersion de cire selon au moins l'une quelconque des revendications 1 à 9, **caractérisée en ce qu'**elle contient en outre au moins un polymère, de préférence en une quantité de 0,01 à 5,0 % en poids, par rapport à la dispersion de cire.

11. Dispersion de cire selon au moins l'une quelconque des revendications 1 à 10, **caractérisée en ce que** le polymère est choisi dans le groupe constitué par les polyacrylates, les polysaccharides, les polyacrylamides ou un mélange quelconque de ces polymères.

12. Dispersion de cire selon au moins l'une quelconque des revendications 1 à 11, **caractérisée en ce que** la phase cireuse contient en outre au moins un agent actif.

13. Dispersion de cire selon au moins l'une quelconque des revendications 1 à 12, **caractérisée en ce qu'**elle contient en outre au moins un agent de rétention de l'humidité.

14. Procédé de fabrication d'une dispersion de cire ayant une taille de particule moyenne (par diffraction de Fraunhofer) de 0,5 à 100 µm, **caractérisé en ce qu'**une pré-émulsion de la phase cireuse avec une phase aqueuse est fabriquée, et cette pré-émulsion, qui présente une température supérieure à la plage de fusion des cires, est introduite sous pression dans une phase aqueuse contenant un polymère, qui présente une température de 1 à 30 °C.

15. Procédé selon la revendication 14, **caractérisé en ce que** la pré-émulsion est homogénéisée au moins une fois dans une étape intermédiaire avant d'être introduite dans la phase aqueuse.

16. Procédé selon au moins l'une quelconque des revendications 14 à 15, **caractérisé en ce que** la pré-émulsion est refroidie par un échangeur de chaleur avant l'introduction dans la phase aqueuse.

17. Procédé selon au moins l'une quelconque des revendications 14 à 16, **caractérisé en ce que** la pré-émulsion contient également un polymère.

18. Procédé selon au moins l'une quelconque des revendications 14 à 17, **caractérisé en ce que** le polymère est choisi dans le groupe constitué par les polyacrylates, les polysaccharides, les polyacrylamides ou un mélange quelconque de ces substances.

19. Procédé de fabrication d'une dispersion de cire selon au moins l'une quelconque des revendications 14 à 18, **caractérisé en ce que** la pré-émulsion est injectée sous pression par une buse dans la phase aqueuse.

20. Procédé selon au moins l'une quelconque des revendications 14 à 19, **caractérisé en ce que** la phase cireuse contient au moins un composant huileux ou cireux choisi parmi les éthers de dialkyl(ène), les carbonates de dialkyl(ène), les acides dicarboxyliques ou les alcools hydroxy-gras ou un mélange quelconque de ces substances et au moins un émulsifiant.

21. Procédé selon au moins l'une quelconque des revendications 14 à 20, **caractérisé en ce que** la phase cireuse présente un point de fusion supérieur à 25 °C.

22. Procédé selon au moins l'une quelconque des revendications 14 à 21, **caractérisé en ce que** la dispersion de cire résultante comprend
(a) 10 à 75 % en poids d'une phase cireuse et
(b) 25 à 90 % en poids d'une phase aqueuse,
par rapport à la composition totale.

23. Utilisation d'une dispersion de cire selon l'une quelconque des revendications 1 à 13 en tant qu'agent de soin du corps ou pour la fabrication d'agents de soin du corps.
